# NEW EUROPEAN PATENT SPECIFICATION

(11) **EP 0 424 428 B2**
(45) Date of publication and mention of the opposition decision: **21.10.1998**
(45) Mention of the grant of the patent: 07.09.1994
(21) Application number: 89907782.0
(22) Date of filing: 26.06.1989
(51) Int. Cl.: G01N 33/68, G01N 33/53

(54) **METHOD OF MONITORING COLLAGEN DEGRADATION**
VERFAHREN ZUR ÜBERWACHUNG DES KOLLAGENABBAUS
PROCEDE DE CONTROLE DE DEGRADATION DE COLLAGENE

(30) Priority: 25.06.1988 GB 8815174
(43) Date of publication of application: 02.05.1991
(73) Proprietor: The Rowett Research Institute, Aberdeen AB2 9SB Scotland (GB)
(72) Inventor: ROBINS, Simon, Peter, Aberdeenshire AB5 9QW (GB)
(74) Representative: Hallybone, Huw George
(86) International application number: GB8900715
(87) International publication number: WO8912824

(56) References cited:
- EP-B- 394 296
- WO-A-89/04491
- US-A- 5 283 197
- JOURNAL OF BONE & MINERAL RESEARCH, vol. 3, suppl. I, June 1988, Mary Ann Liebert Inc.; D. EYRE et al., no. 565#
- ANNUAL REVIEWS OF BIOCHEMISTRY, vol. 53, 1984, Annual Reviews Inc.; D. EYRE et al., pp. 717-748#
- ANALYTICAL CHEMISTRY Vol. 169, 1988, D. BLACK et al., "Quantitative Analysis of the Pyridinium Crosslinks of Collagen in Urine Using Ion-Paired Reversed-Phase High-Performance Liquid Chromatography", pages 197-203
- ANNALS OF THE RHEUMATIC DISEASES Vol. 45, 1986, S.P. ROBINS et al., "Measurement of the Cross Linking Compound, Pyridinoline, in Urine as an Index of Collagen Degradation in Joint Disease", pages 969-973
- CHEMICAL ABSTRACTS Vol. 105, No. 7, 18 August 1986, (Columbus, Ohio, US), SCHUPPAN D. et al., "Radioimmunoassay for the Carboxy-terminal Cross-linking Domain of Type IV (Basement Membrane) Procollagen in Body Fluids. Characterization and Application to Collagen Type IV Metabolism in Fibrotic Liver Disease", page 463, Abstract 58896z, & J. CLIN. INVEST., 1986, 78(1), 241-8 (ENG)
- CHEMICAL ABSTRACTS Vol. 99, No. 19, 7 November 1983, (Columbus, Ohio, US), FUJIMOTO DAISABURO et al., "Analysis of Pyridinoline, a Cross-linking Compound of Collagen Fibers, in Human Urine", page 446, Abstract 156368f, & J. BIOCHEM.,(TOKYO), 1983, 94(4), 1133-6 (ENG)
- BIOCHEM. J. Vol. 207, 1982, S.P. ROBINS, "An Enzyme-linked Immunoassay for the Collagen Cross-link Pyridinoline", pages 617-620
- CARDIOVASCULAR RESEARCH Vol. 21, 1987, M.A. WHITTLE et al., "Biochemical Investigation of Possible Lesions in Human Aorta that Predispose to Dissecting Aneurysms: Pyridinoline Crosslinks", pages 161-168
- ANALYTICAL BIOCHEMISTRY Vol. 147, 1985, ZEENAT GUNJA-SMITH, "An Enzyme-linked Immunosorbent Assay to Quantitate the Elastin Crosslink Desmosine in Tissue and Urine Samples", pages 258-264
- SCOTTISH MEDICAL JOURNAL Vol. 33, (1988):4, SCOTTISH SOCIETY FOR EXPERIMENTAL MEDICINE, Meeting on 13th May 1988, page 317
- J. Biochem 215, 1983; S Robbins, p.167-173
- J.Biochemistry 94, 1983; Fujimoto et al., p.1133-1136
- Biophys, Acta 229, 1971;Pinnell et al., p.119-122
- Calcif. Tissue Int. 42, 1988; Moro et al., p87-90
- Calcif. Tissue Int. 44, 1989; Black et al., p.343-347

## Description

This invention relates to a method of monitoring collagen degradation as a diagnostic aid in relation particularly to osteoporosis and rheumatoid arthntis.

Collagen is present in various form in all tissue. It has been shown (Fujimoto et al., (1978) Biochemistry, Biophysics Research Communication vol. 84, 52-57) that collagen has the form of amino acid chains crosslinked by pyridinoline. The pyridinium crosslinks are formed from three hydroxylysine residues, two from the terminal (non-helical) peptides of the collagen molecule that are enzymically converted to aldehydes before reaction and a third hydroxylysine situated in the helical portion of a neighbouring collagen molecule. Robins et al (Annals of the Rheumatic Diseases 1986, 45, 969-973) have described a technique for measurement of pyridinoline in urine by use of an antibody specific to pyridinoline and detected by enzyme-linked immunosorbent assay (ELISA). This technique, however gives a measure only of hydroxylysyl pyridinoline in the sample, and does not recognise lysyl deoxypyridinoline. The former is present in bone and tissue, and the latter in bone only. Thus the technique may indicate the occurrence of collagen degradation, indicating the presence of degenerative disease, but without indicating the type of tissue concerned.

WO 89/04491. published 18.05.39, describes a method for measuring cone resorption, in which peptide fragments containing a 3-hydroxypyridinium crosslink are measured in inhydrolyzed urine.

The present invention is based on the recognition that lysyl and hydroxylysyl pyridinoline are present in biological fluid such as urine, attached to fragments of the original amino acid chains, or to sugars. However, there is no certainty as to where the original chains will have broken. In virtually all cases, however, sufficient amino acids will be present to identify the type of tissue from which the particular collagen derived.

According to the present invention there is a method of monitoring bone collagen resorption in a human subject comprising:
obtaining a urine sample from the subject determining the amount of a peptide-free glycosylated hydroxylysyl pyridinoline in the sample, with such in a non-hydrolysed form, and
using the amount of peptide-free glycosylated hydroxylysyl pyridinoline determined as an indicator of a bone-collagen degradation.

Preferably the sugar is linked to pyridinoline by glycosylation with galactose or with glucose and galactose.

The collagen may suitably be associated with bone or cartilage.

Embodiments of the invention will now be described in further detail by way of example.

Collagen has the general structure: 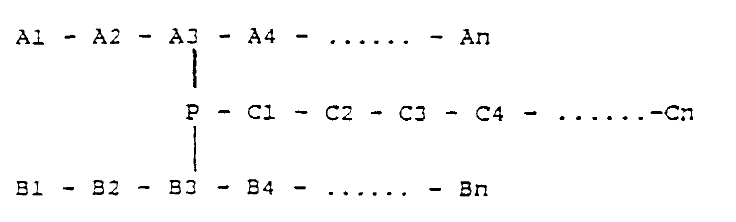 where A's, B's and C's are amino acids and P is lysyl or hydroxylysyl pyridinoline or glycosylated hydroxylysyl pyridinoline.

Hydroxylysine residues in the helix are glycosylated by addition of sugar groups at their hydroxyl group. Where formation of the pyridinoline (Pyd) crosslink involves a glycosylated hydroxylysine, a glycosylated crosslink will be produced. The crosslink analogue, lysyl-pyridinoline, formed by reaction with a lysine residue in the helix cannot form any glycosylated derivatives as it lacks the side-chain hydroxyl group.

There are two types of glycosylation, either galactose (Gal) alone or the disaccharide, glucosyl-galactose (Gal.Gic). Thus, there are two possible forms of peptide-free glycosylated hydroxylysyl pyridinoline as shown below, Pyd-Gal and Pyd-Gal.Glc.

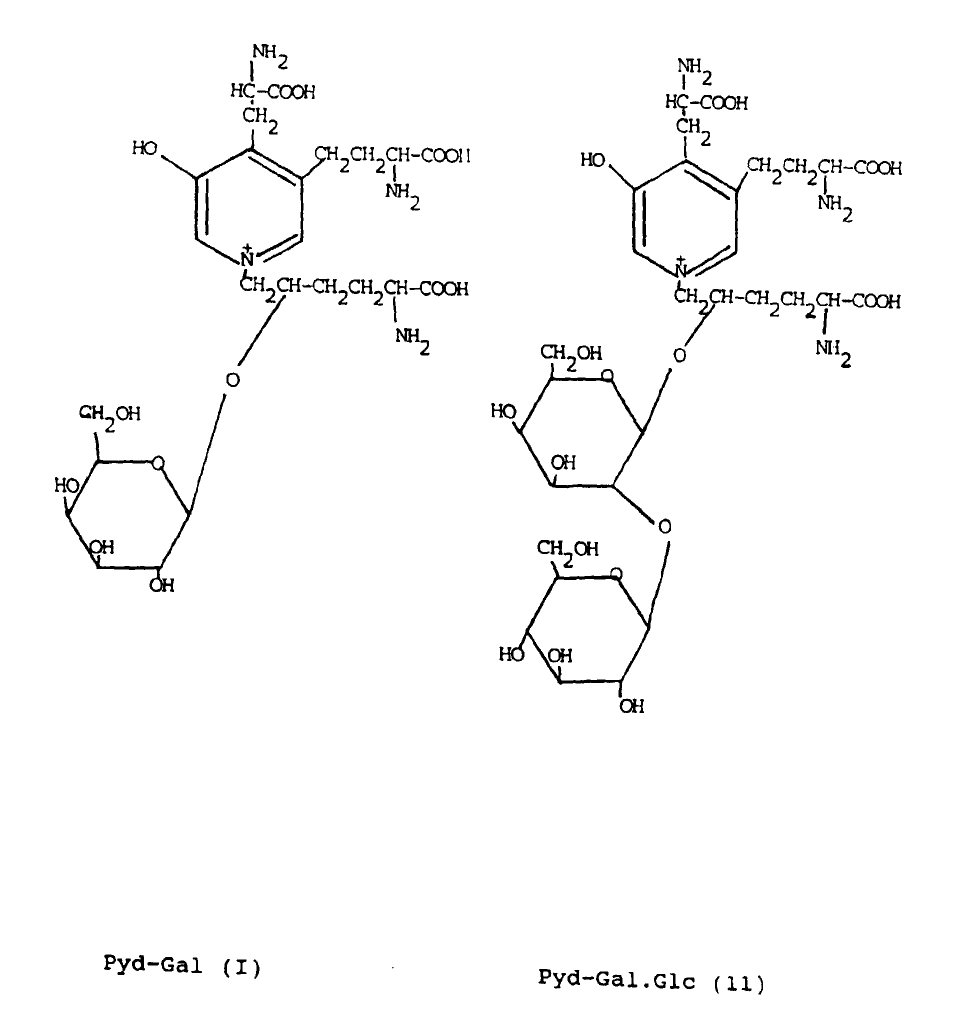

The relative proportion of mono- to di-saccharide derivatives of hydroxylysine varies with different tissues. For the main tissues of interest (i.e. those that contain pyridinoline) cartilage contains almost entirely Gal.Glc, whereas in bone collagen the monosaccharide predominates.

Both the mono- and di-saccharide derivatives of pyridinoline (structures I and II) have been isolated from human urine and identified. These components are present normally in urine but have been shown to be present in increased amounts in various bone disorders and in arthritic disease.

The main points of interest in these findings are:
1. The components are present in urine without hydrolytic treatment and an assay procedure would therefore be applicable directly to urine.
2. Assays of the two components will give some tissue-specific information on collagen breakdown. Measurement of Pyd-Gal will provide an index of bone collagen resorption: Pyd-Gal.Glc amounts in urine are primarily indicative of bone or cartilage degradation, and the relative amounts of Pyd-Gal and Pyd-Gal.Glc will provide information on the relative extent of bone and cartilage degradation.
3. The presence of the sugar groups may increase the antigenicity (ease with which good antibodies can be raised) of the components. The sugar portions will form part of the antibody recognition sites so that antibodies specific for each structure can be produced.

Two types of tissue of particular interest are bone and cartilage. The presence of degradation of bone collagen alone is an indicator of osteoporosis and other bone disorders, while the presence of degradation of both bone collagen and cartilage collagen is an indicator of arthritic disorders or diseases.

In general, antibodies can be obtained by following the steps:
1. Identify a particular X of interest in biological fluid.
2. Isolate X.
3. Attach X to a suitable protein.
4. Inject the product of 3 into a host animal and raise antibody.
5. Use this antibody in ELISA or other suitable assay technique.

More specific examples of the invention will now be given.

Schemes 1 and 2 summarise the strategies for isolating crosslink-containing fragments from urine and give details of the components being isolated. Variations on these Schemes are detailed below.

### EXAMPLE 1 (comparative)

### Method

a. Urine samples containing high concentrations of total pyridinium crosslinks were collected from patients with disorders involving either increased bone turnover (hyperparathyroidism) or increased cartilage and bone degradation (rheumatoid arthritis).
b. A total of 1.0-1.51 of urine was freeze-dried, redissolved in 0.2 M acetic acid and was subjected to gel filtration chromatography in batches using a 2.6 x 140cm column of Biogel® P2.
c. Selected fractions containing pyridinium crosslink derivatives were re-chromatographed by reversed-phase HPLC using a C₁₈ support and elution with a mobile phase containing acetonitrile. The peptides were further purified by ion-exchange HPLC using DEAE- and SP-5PW columns.
d. The isolated components were characterized by fast atom bombardment mass spectrometry and amino acid sequence analysis; glycosylation sites were identified by gas-liquid chromatography of alkali hydrolysates of the peptides.
e. With a knowledge of the amino acid sequences around the crosslink sites in bone and cartilage, the tissues of origin of the peptides isolated from urine were established. A number of three peptides containing the core sequence representative of different tissues was chosen for raising antibodies.
f. Each of the purified peptides (1 µmol) were covalently attached to ovalbumin (0.25 µmol) using N-ethyl-N'-(3-dimethylaminopropyl) carbodiimide hydrochloride.
g. Balb/c mice were immunized with the ovalbumin conjugates and monoclonal antibodies were produced after fusion with Ag8.653 myeloma cells. Positive clones were detected by EUSA using microtube plates coated with the peptides attached to gelatin by reaction with a different carbodiimide reagent N-cyclohexyl-N'-2-(4'-methylmorpholinium) ethyl carbodiimide-p-toluene sulphonate.

The monocional antibodies so produced may be used in immunoassay tests for the diagnosis and monitoring of various types of bone disorders and degenerative joint diseases.

### Example 2

### Method

- Step 1.: Urine (300ml), concentrated to one tenth its original volume, was chromatographed on a column (3.0 x 100cm ) of sephadex G25M with 0.2M-acetic acid as eluant The fractions between 540 and 630ml (Vₑ/V₀=1.6-1.9) that contained approx. 60% of the characteristic pyridinium crosslink fluorescence were pooled and freeze-dried.
- Step 2.: The fraction from Step 1 (280mg) was rechromatographed on a column (1.7 x 140cm) of Sephadex® G10 eluted with O.2M-acetic acid to separate the crosslinking components from amino acids and other small-molecular weight material. The fractions were monitored by fluorescence (Ex 295nm; Emm 400nm) and material that eluted between 105 and 121ml (Vₑ/Vₒ = 1.1-1.3) was pooled and evaporated to dryness.
- Step 3.: The fraction from Step 2 was subject to cation-exchange chromatography on a column (0.9 x 15cm) of sulphonated polystyrene resin beads (5µ) crosslinked (8%) with divinyl benzene (Locarte Co Ltd. London; Cat No LA 48/08) run at 56°C and eluted with 67mM sodium citrate buffer. pH 3.84. Fractions of 5ml were collected and appropriate fractions were desalted on a column (1.7 x 50cm) of Sephadex G10 and freeze-dried.
In this system, Pyd-Gal (I) eluted between 91 and 105min and pyd-Gal.Glc (II) eluted between 41 and 50min.
Final purification of I and II was reversed-phase HPLC on a column (0.9 x 25cm) of Rosil C₁₈ packing (3µ) eluted with 0.1% heptafluorobutyric acid and a linear gradient from 15 to 30% acetonitrile over 30 min. (I) and (II) eluted at 17.3 and 16.9min respectively.

### CHARACTERISATION

### 1. Hydrolysis characteristics

Mild acid hydrolysis (0.1 M HC1 at 108 C for 12h) converted Pyd-Gal.Glc (II) completely into a mixture of Pyd-Gal (I) and pyridinoline (Pyd). Hydrolysis of Pyd-Gal (I) in 2M HC1 at 108 °C for 8h effected complete conversion of this component to Pyd. This behaviour is characteristic for these types of compound and has been noted previously for the interconversion of hydroxylysine glycosides to hydroxylysine.

### 2. Composition

On hydrolysis with strong mineral acid, both compounds (I) and (II) produced Pyd with no other amino acids detected in the hydrolysates. The carbohydrate compositions were determined by gas-liquid chromatography of methylated derivatives, after hydrolysis in 2M H₂SO₄ and reduction with sodium borohydride. Based on these results and determination of the amounts of crosslink by HPLC, the molar ratios were:
- Component I: Pyridinoline (1.0); Galactose (0.9)
- Component II: Pyridinoline (1.0): Galactose (0.8); Glucose (0.9)

The results above therefore confirm that the structures of compounds I and II are as shown earlier.

In addition to the above, an associated derivative, termed 'X', that eluted between 184 and 195min at Step 3, has been isolated. This component contains Pyd and is increased in amount in patients with bone disorders.

Immunoassays for these components will be developed precisely as described for the peptides by conjugation to ovalbumin through their amino or carboxyl groups.

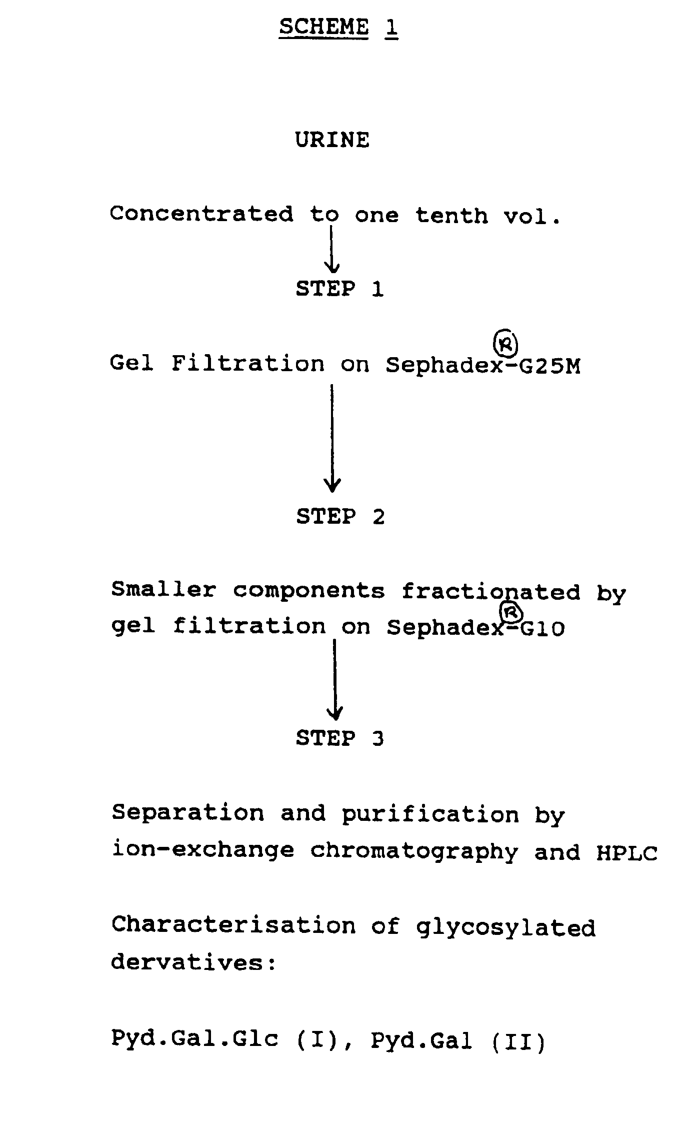

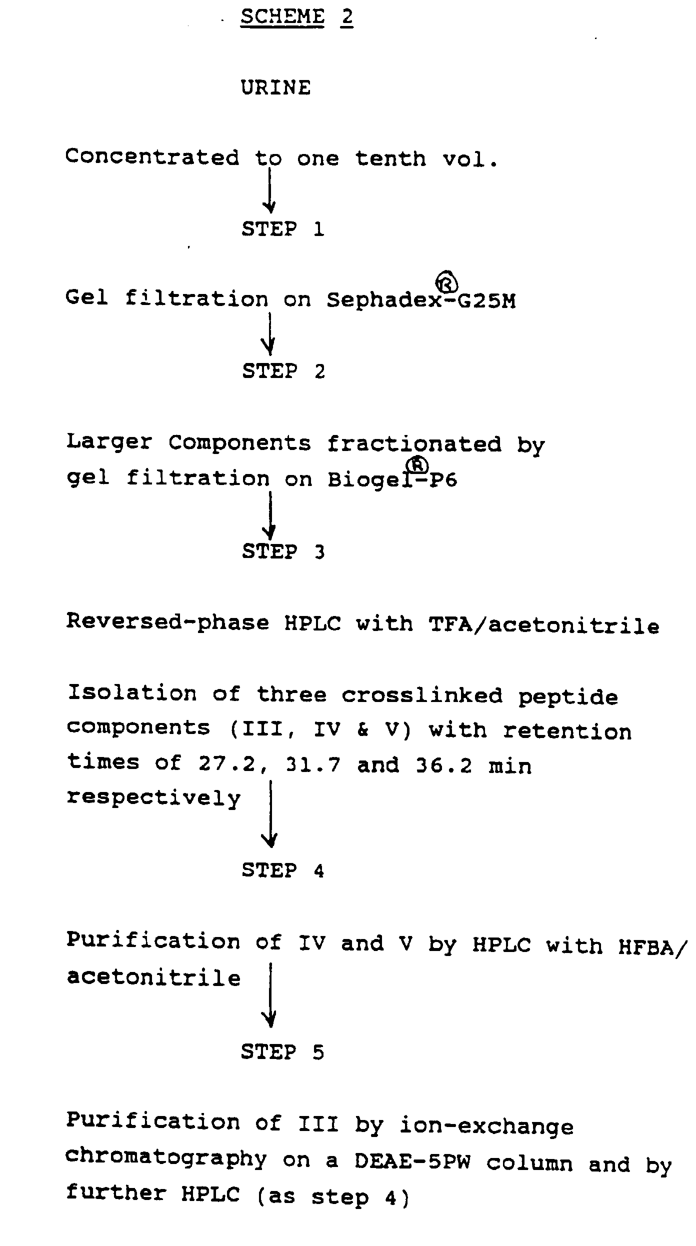

## Claims

1. A method of monitoring bone collagen resorption in a human subject comprising:
obtaining a urine sample from the subject, determining the amount of a peptide-free glycosylated hydroxylysyl pyridinoline in the sample, with such in a non-hydrolysed form, and
using the amount of peptide-free glycosylated hydroxylysyl pyridinoline determined as an indicator of a bone-collagen degradation.

2. The method of Claim 1, wherein the peptide-free glycosylated hydroxylysyl pyridinoline is galactosyl pyridinoline.

3. The method of Claim 1, wherein the peptide-free glycosylated hydroxylysyl pyridinoline is glucosyl galactosyl pyridinoline.

## Patentansprüche

1. Verfahren zur Überwachung der Resorption bzw. des Abbaus von Knochencollagen bei einem Patienten, umfassend :
Gewinnung einer Urinprobe von dem Patienten,
Bestimmung der Menge an einem peptidfreien glycosylierten Hydroxylysylpyridinolin in der Probe mit einem solchen in einer nichthydrolysierten Form und
Anwendung der bestimmten Menge an peptidfreiem glycosyliertem Hydroxylysyl-pyridinolin als Indikator für einen Abbau von Knochencollagen.

2. Verfahren nach Anspruch 1, wobei das peptidfreie glycosylierte Hydroxylysyl-pyridinolin ein Galactosyl-pyridinolin ist.

3. Verfahren nach Anspruch 1, wobei das peptidfreie glycosylierte Hydroxylysyl-pyridinolin ein Glucosyl-galactosyl-pyridinolin ist.

## Revendications

1. Procédé pour contrôler la résorption du collagène osseux chez un sujet humain, ledit procédé comprenant :
l'obtention d'un échantillon d'urine du sujet,
la détermination de la quantité d'une hydroxylysyl-pyridinoline glycosylée exempte de peptide présente dans l'échantillon sous une forme non-hydrolysée, et
l'utilisation de la quantité d'hydroxylysyl-pyridinoline glycosylée exempte de peptide, déterminée en tant qu'indicateur de dégradation du collagène osseux.

2. Procédé suivant la revendication 1, dans lequel l'hydroxylysyl-pyridinoline glycosylée exempte de peptide est la galactosyl-pyridinoline.

3. Procédé suivant la revendication 1, dans lequel l'hydroxylysyl-pyridinoline glycosylée exempte de peptide est la glucosyl-galactosyl-pyridinoline.
